# EUROPEAN PATENT SPECIFICATION

(11) **EP 0 866 678 B1**
(45) Date of publication and mention of the grant of the patent: **12.09.2007**
(21) Application number: 96944296.1
(22) Date of filing: 13.12.1996
(51) Int. Cl.: A61F 2/06

(54) **KINK-RESISTANT STENT GRAFT**
KNICKBESTÄNDIGES STENT-TRANSPLANTAT
STENT A GREFFER RESISTANT AUX PLIS

(30) Priority: 14.12.1995 US 572548
(43) Date of publication of application: 30.09.1998
(73) Proprietor: Gore Enterprise Holdings, Inc., Newark, DE 19714 (US)
(72) Inventor: MARTIN, Gerald, Ray, Flagstaff, AZ 86004 (US); LAU, Lilip, Sunnyvale, CA 94087 (US); STONEBROOK, Scott, N., Flagstaff, AZ 86001 (US); LAM, Sharon, San Jose, CA 94087 (US); THORNTON, Troy, San Francisco, CA 94107 (US)
(74) Representative: Shanks, Andrew
(86) International application number: PCT/US1996/019669
(87) International publication number: WO 1997/021403

(56) References cited:
- WO-A-95/26695
- US-A- 5 423 849
- US-A- 5 549 663

## Description

### FIELD OF THE INVENTION

This invention relates generally to implants for repairing ducts and passageways in the body. More specifically, the invention relates to an expandable stent-graft.

### BACKGROUND OF THE INVENTION

Treatment or isolation of vascular aneurysms or of vessel walls which have been thinned or thickened by disease has traditionally been performed via surgical bypassing with vascular grafts. Shortcomings of this procedure include the morbidity and mortality associated with surgery, long recovery times after surgery, and the high incidence of repeat intervention needed due to limitations of the graft or of the procedure.

Vessels thickened by disease are currently sometimes treated less invasively with intralumenal stents that mechanically hold these vessels open either subsequent to or as an adjunct to a balloon angioplasty procedure. Shortcomings of current stents include the use of highly thrombogenic materials (stainless steels, tantalum) which are exposed to blood, the general failure of these materials to attract and support functional endothelium, the irregular stent/vessel surface that causes unnatural blood flow patterns, and the mismatch of mechanical compliance and flexibility between the vessel and the stent.

Various attempts have been made to provide a nonthrombogenic blood-carrying conduit. Pinchuk, in U.S. Pat. Nos. 5,019,090, 5,092,877, and 5,163,958, discloses a helically wrapped spring stent. The Pinchuk '958 patent appears to disclose the use of a pyrolytic carbon layer on the surface of the stent to present a porous surface of improved antithrombogenic properties.

U.S. Patent No. 5,123,917, to Lee, discloses an expandable vascular graft having a flexible cylindrical inner tubing and a number of "scaffold members" which are expandable, ring-like, and provide circumferential rigidity to the graft. The scaffold members are deployed by deforming them beyond their plastic limit using, e.g., an angioplasty balloon.

A variety of stent-graft designs also have been developed to improve upon simple stent configurations. Perhaps the most widely known stent-graft is shown in Ersek, U.S. Pat. No. 3,657,744. Ersek shows a system for deploying expandable, plastically deformable stents of metal mesh having an attached graft through the use of an expansion tool.

Palmaz describes a variety of expandable intraluminal vascular grafts in a sequence of patents: U.S. Patent Nos. 4,733,665; 4,739,762; 4,776,337; and 5,102,417. The Palmaz '665 patent suggests grafts (which also function as stents) that are expanded using angioplasty balloons. The grafts are variously a wire mesh tube or of a plurality of thin bars fixedly secured to each other. The devices are installed, e.g., using an angioplasty balloon and consequently are not seen to be self-expanding. The Palmaz '762 and '337 patents appear to suggest the use of thin-walled, biologically inert materials on the outer periphery of the earlier-described stents. Finally, the Palmaz'417 patent describes the use of multiple stent sections each flexibly connected to its neighbor.

Rhodes, U.S. Pat. No. 5, 122, 154, shows an expandable stent-graft made to be expanded using a balloon catheter. The stent is a sequence of ring-like members formed of links spaced apart along the graft. The graft is a sleeve of a material such as an expanded polyfluorocarbon (e.g., GORE-TEX® or IMPRA™.

Schatz, U.S. Pat. No. 5,195,984, shows an expandable intraluminal stent and graft related in concept to the Palmaz patents discussed above. Schatz discusses, in addition, the use of flexibly-connecting vascular grafts which contain several of the Palmaz stent rings to allow flexibility of the overall structure in following curving body lumen.

Cragg, "Percutaneous Femoropopliteal Graft Placement", Radiology, vol. 187, no. 3, pp. 643-648 (1993), shows a stent-graft of a self-expanding, nitinol, zig-zag, helically wound stent having a section of polytetrafluoroethylene tubing sewed to the interior of the stent.

Cragg (European Patent 0, 556, 850) discloses an intraluminal stent made up of a continuous helix of zig-zag wire and having loops at each apex of the zig-zags. Those loops on the adjacent apexes are individually tied together to form diamond-shaped openings among the wires. The stent may be made of a metal such as nitinol (col. 3, lines 15 - 25 and col. 4, lines 42+) and may be associated with a "polytetrafluoroethylene (PTFE), Dacron, or any other suitable biocompatible material". Those biocompatible materials may be inside the stent (col. 3, lines 52+) or outside the stent (col. 4, lines 6+).

WO 93/13825 to Maeda et al. discloses a self-expanding stent having a wire bent into an elongated zig-zag pattern and helically wound about a tubular shape interconnected with a filament. A sleeve may be attached to the outer or inners surface of the stent.

PCT application publication WO 95/05132 discloses a stent-graft with a tubular diametrically adjustable stent.

WO 95/26695 relates to a self-expandable stent-graft comprising a stent, a tubular graft member and a filament as a coupling member. The device is foldable and may be delivered with a catheter or via surgical or other suitable techniques.

There is a need for an alternate stent-graft construction that exhibits excellent kink resistance and flexibility.

### SUMMARY OF THE INVENTION

The present invention involves a stent-graft including a stent member having an inner surface and an outer surface, a generally tubular graft member and a coupling member that couples the stent member to the graft member. The coupling member is in the form of a ribbon, covers only a portion of at least one of the inner or outer surface of the stent member and secures the stent member and graft member to one another. Alternatively, the coupling member can be described as interconnecting less than entirely the inner or outer surface of the stent member to the graft member.

With this construction, regions of the stent member do not interface with the coupling member. This is believed to advantageously reduce shear stresses between the stent member and the coupling member when the stent-graft undergoes bending so that the tearing of the coupling and/or graft member can be minimized or eliminated. It is also believed that this arrangement minimizes the likelihood of delamination between the coupling member and the graft. If delamination were to occur, the inner portion of the stent-graft could perceivably collapse into the vessel lumen and interfere with desired blood flow. Thus, the stent-graft is believed to be capable of conforming to curves in a blood vessel lumen with minimal risk of tearing the graft or coupling member, or delamination between the stent and graft members.

According to another aspect of the invention, the coupling member is secured to the graft member without sutures. When the graft member is placed within the stent member, for example, this arrangement eliminates the need for having sutures extend into the lumen formed by the graft member and possibly interfere with blood flow. Another benefit of this arrangement as compared to suturing the stent to the graft member is that suture holes need not be placed in the graft which could adversely affect its integrity. The coupling member may be thermally or adhesively bonded to the graft member.

The coupling member preferably has a generally broad or flat working surface as compared to filament or thread-like structures such as sutures. As noted above, the coupling member is in the form of a ribbon. This configuration advantageously increases potential bonding surface area between the coupling member and the graft member to enhance the integrity of the bond therebetween. The increased bonding surface area also may facilitate minimizing the thickness of the coupling member so that the stent-graft lumen volume and blood flow dynamics therein can be optimized. For example, a thicker coupling member would increase the overall stent-graft thickness which can cause an undesirable lumen diameter reduction at the transition where the vessel lumen interfaces the inlet of the stent-graft. This, in turn, can result in undesirable turbulent flow which possibly can lead to complications such as thrombosis.

According to a preferred embodiment of the invention, the coupling member is arranged in a helical configuration with multiple turns. Each of a number of the coupling member turns is spaced from the turn(s) adjacent thereto.

With this construction, a generally uniform distribution of coupling member-free stress relief zones may be achieved. Elastic wrinkling in the graft member may occur in those zones so that the graft member can absorb stress when bent along its longitudinal axis, for example, and resist kinking.

According to a preferred stent member construction for use with the stent-graft of the present invention, at least a portion of the stent member includes undulations and is arranged in a helical configuration with multiple turns. Each stent member undulation includes an apex and an open base portion. The apexes and base portions are configured so as not to restrain movement of one apex into the undulation in an adjacent turn and substantially in-phase therewith when the stent-graft is bent or compressed. This is believed to facilitate undulation movement during bending or compression and minimize the likelihood of stress build-up that may cause kinking. The coupling member typically covers a substantial portion of each undulation so as to minimize the likelihood of the stent member apexes bending away from the graft member and interfering with the environment or tether line which may be used to maintain the stent-graft in a folded state before deployment. The coupling member also may be positioned adjacent to the apexes to minimize the likelihood of such apex movement.

According to another aspect of the invention, the end portions of the stent-member also may be enveloped between the coupling member or discrete coupling members and the graft member. This prevents the terminal portions of the stent and graft members from significantly moving away from one another. For example, when the stent member is external to the graft member, the terminal graft portions may flap away from the stent member and possibly interfere with blood flow if the terminal coupling portions were not present.

The above is a brief description of some deficiencies in the prior art and advantages of the present invention. Other features, advantages, and embodiments of the invention will be apparent to those skilled in the art from the following description, accompanying drawings and appended claims.

### BRIEF DESCRIPTION OF THE DRAWINGS

Fig. 1A is a perspective view of a stent-graft constructed in accordance with the principles of the present invention.
Fig. 1B is an enlarged perspective view of a mid-portion of the stent-graft shown in Fig. 1A.
Fig. 2 is a side view of an enlarged portion of the stent-graft shown in Fig. 1A.
Fig. 3A is a diagrammatic representation of a transverse section of the stent-graft of Fig. 1 prior to the coupling and graft members being secured to one another.
Fig. 3B is an enlarged portion of the section shown in Fig. 3A after the coupling and graft members have been secured to one another.
Fig. 4 illustrates the stent-graft of Figs. 1A & 1B under longitudinal, axial compression.
Fig. 5 is a sectional view of the stent-graft of Figs. 1A & 1B taken along line 5-5 in Fig. 4.
Fig. 6 diagrammatically shows a portion of the stent-graft of Figs. 1A & 1B bent along its longitudinal axis.
Fig. 7 is a perspective view of another embodiment of the stent-graft of the present invention having an alternate stent to graft coupling configuration.
Fig. 8 is a side view of an enlarged portion of the stent-graft shown in Fig. 7.
Fig. 9 is a perspective view of a further embodiment of the stent-graft of the present invention having yet another stent to graft coupling.
Fig. 10 is a side view of an enlarged portion of the stent-graft shown in Fig. 9.
Fig. 11 is a partial view of the stent-graft of Fig. 1A showing an end portion of the device.
Fig. 12 is an abstracted portion of a suitable stent and shows the concept of torsion on a portion of that stent.
Fig. 13A diagrammatically shows a stent-graft of the present invention with flared ends (the coupling tape drawn back to more clearly show the helically wound undulating stent configuration).
Fig. 13B diagrammatically shows a further stent-member construction for supporting the graft member.
Figs. 14A, 14B, 14C, 14D, and 14E are plan views of unrolled stent forms suitable for use in the invention.
Figs. 15A, 15C, and 15E show procedures for folding the stent-grafts. Figs. 15B, 15D, and 15F show the corresponding folded stent-grafts.
Figs. 16A. 16B and 16C diagrammatically show a procedure for deploying the stent-grafts using an external sleeve.
Figs. 17A and 18A are partial perspective views of folded stent-grafts. Figs. 17B, 17C, 18B, and 18C are end views of the stent-grafts shown in Figs. 17A and 18A in folded and open states.
Figs. 19A, 19B and 19C diagrammatically show a procedure for deploying the stent-grafts shown in Figs. 17A-17C and 18A-18C using a tether wire.
Fig. 20 shows an enlarged view of a stent fold line using one sack knot arrangement in the slip line.
Fig. 21 is a diagrammatic perspective view of a folded stent-graft held in position by a tether line and sack knot as illustrated in Fig. 20.
Fig. 22 shows an enlarged view of a stent fold line using another sack knot arrangement in the slip line.
Figs. 23, 24, 25 and 26 are diagrammatic sequential illustrations of a further deployment procedure.

### DESCRIPTION OF THE PREFERRED EMBODIMENTS

Referring to the drawings in detail wherein like numbers indicate like elements, an expandable stent-graft **2** is shown constructed according to the principles of the present invention. Although particular stent and graft constructions will be described in conjunction with the preferred embodiments, it should be understood that other constructions may be used without departing from the scope of the invention

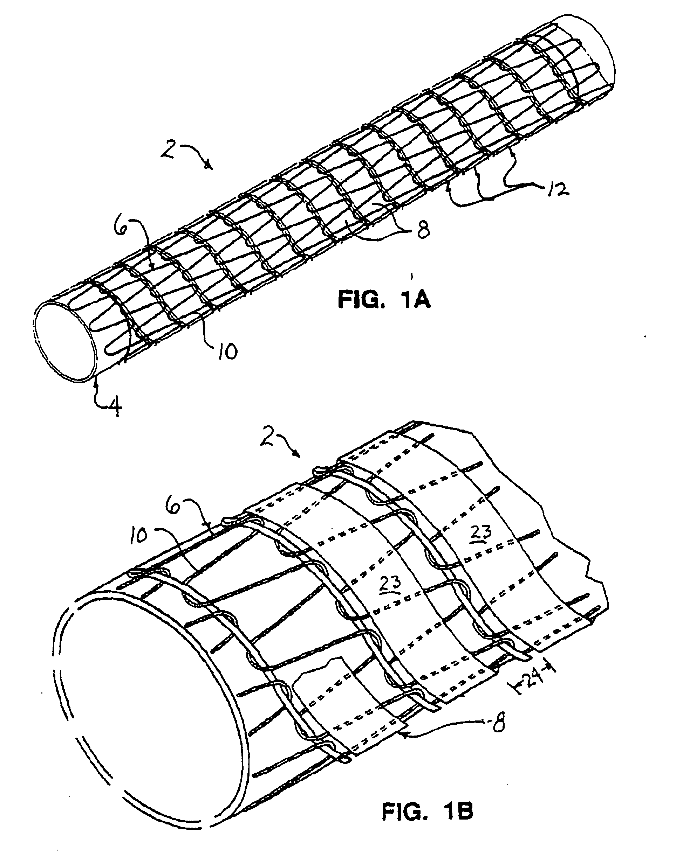

Referring to Figs. 1A and B, stent-graft **2** generally includes a thin-walled tube or graft member **4,** a stent member **6** and a coupling member **8** for coupling the stent and graft members together. Preferably, the stent and graft members are coupled together so that they are generally coaxial.

Expandable stent member **6** is generally cylindrical and comprises a helically arranged undulating member **10** having a plurality of helical turns **12** and preferably comprising nitinol wire. The undulations preferably are aligned so that they are "in-phase" with each other as shown in Figs. 1A and 1B, for example. More specifically, undulating helical member **10** forms a plurality of undulations **14,** each including an apex portion **16** and a base portion **18.** When the undulations are in-phase, apex portions **16** in adjacent helical turns **12** are aligned so that an apex portion **16** may be displaced into a respective base portion **18** of a corresponding undulation in-phase therewith and in an adjacent helical turn.

Once the undulations are aligned so that adjacent undulations in one turn are in-phase with the undulations in the helical turns adjacent thereto, a linking member **20** may be provided to maintain the phased relationship of the undulations during compression and deployment and during bending of the stent member. In the illustrative embodiment, linking member **20** is laced or interwoven between undulations in adjacent turns of the helical member and acquires a helical configuration in being laced as such (See, e.g., Figs. 1-3). Linking member **20** preferably comprises a biocompatible polymeric or metallic material having sufficient flexibility to be readily folded upon itself.

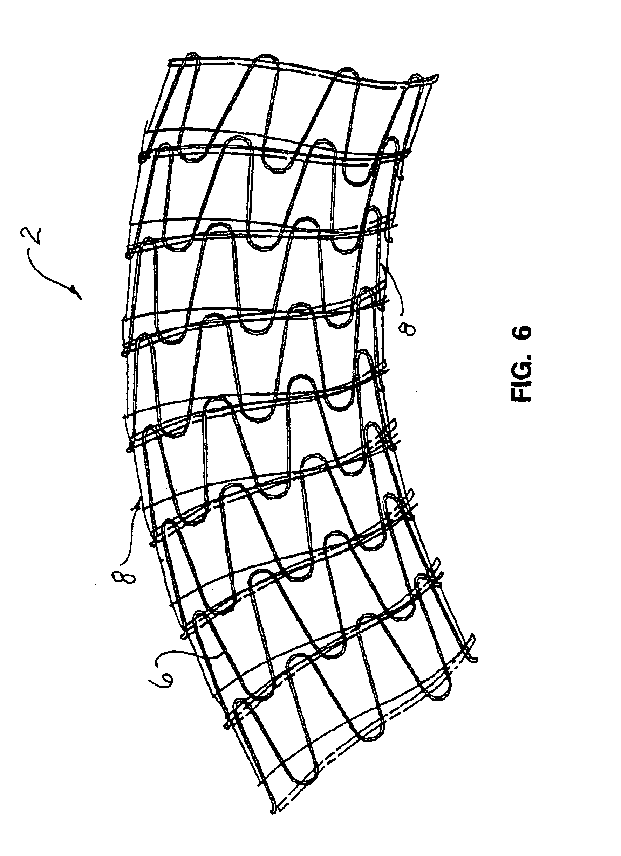

Undulations **14** preferably are unconfined in that they are configured so as not to tend to inhibit the movement of flexible link **20** down between respective torsion arms or lengths **22a** and **22b.** In addition, the undulations preferably are configured and arranged so that a respective apex portion can readily move within a corresponding undulation base portion **18** in-phase therewith. It is believed that this construction minimizes the likelihood of stress build-up, for example, during bending or compression (as depicted in the lower portion of Fig. 6) and, thus, improves the kink resistance of the stent-graft.

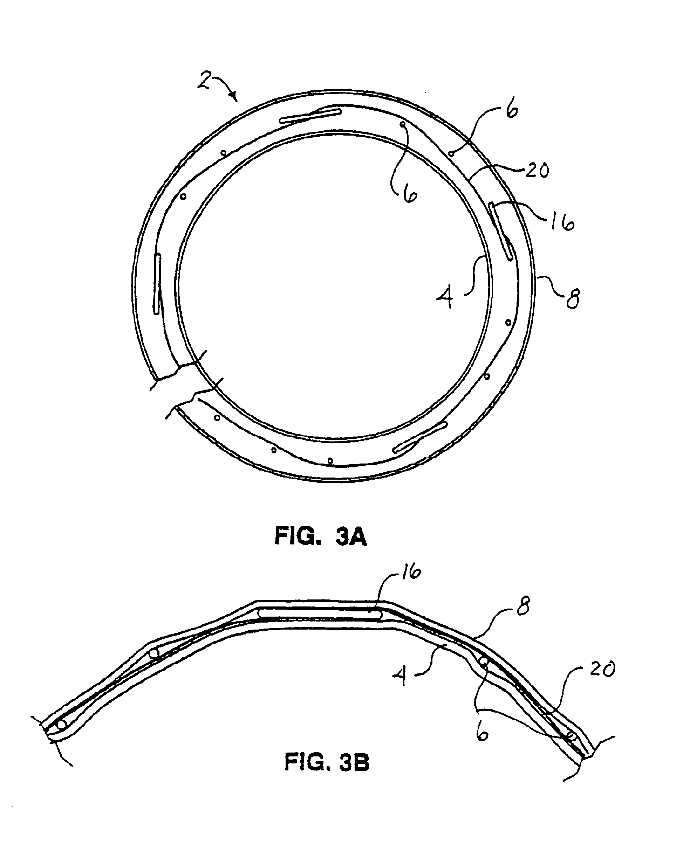

Referring to Figs. 3A and 3B, stent member **6** is disposed between generally tubular graft member **4** and coupling member **8.** The stent member provides a support structure for the graft member to minimize the likelihood of the graft member collapsing during use. Although the graft member may surround the outer surface of the stent member, it preferably is placed within the stent member to provide a relatively smooth (wrinkles may form in the graft member between coupling member turns during compression) intralumenal stent-graft surface as shown in the drawings.

An important aspect of the invention is that the coupling member, which secures the stent member to the graft member, covers only a portion of the stent member. Alternatively, the coupling member can be described as preferably interconnecting less than entirely the inner or outer surface of the stent member to the graft member (e.g., it covers less than all of the outer surface of the stent member when the graft member is positioned inside the stent member). With this construction, regions of the stent member do not interface with the coupling member when the stent-graft is an uncompressed state, for example. This is believed to advantageously reduce sheer stresses between the stent member and the coupling member when the stent-graft undergoes bending or compression, thereby reducing the risk of tearing the graft or coupling member or causing delamination between the stent and graft members.

The coupling member also preferably has a generally broad or flat surface for interfacing with the stent and graft members as compared to filament or thread-like structures such as sutures. This increases potential bonding surface area between the coupling member and the graft member to enhance the structural integrity of the stent-giraft. The increased bonding surface area also facilitates minimizing the thickness of the coupling member. It has been found that a coupling member in the form of a generally flat ribbon or tape as shown in the drawings and designated with reference numeral **8** provides the desired results.

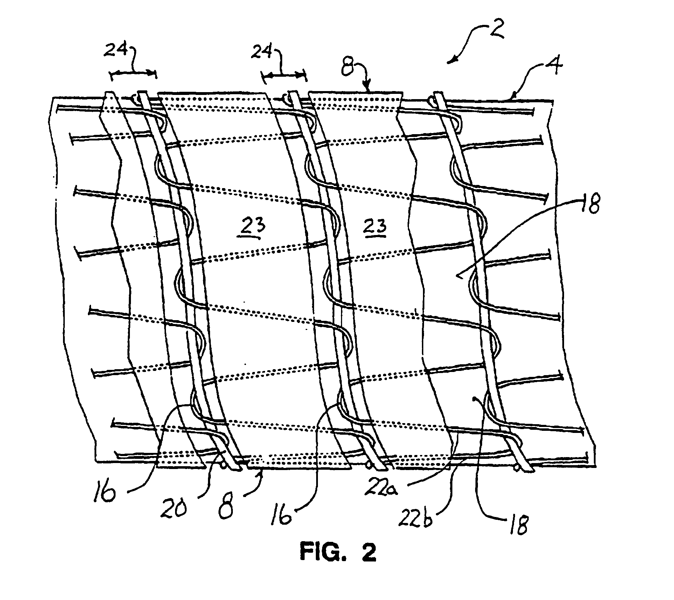

As noted above, coupling member **8** preferably is in the form of a generally flat ribbon or tape having at least one generally flat surface. In addition, coupling member **8** is arranged in a helical configuration according to the preferred embodiments illustrated in the drawings. Referring to Fig. 2, helically arranged coupling member **8** is formed with multiple helical turns **23,** each being spaced from the turns adjacent thereto, thereby forming coupling member-free stress relief zones **24** between adjacent turns. The coupling member also preferably is arranged to provide a generally uniform distribution of stress relief zones **24.** In the illustrated embodiments, coupling member **8** is helically wound around the stent member with its helical turns **23** aligned with the stent member turns **12.** As shown, the coupling member may be constructed with a constant width and arranged with uniform spacing between turns.

Coupling member **8** also preferably covers a substantial portion of each undulation so as to minimize the likelihood of the stent member apexes lifting away from the graft member and interfering with their immediate environment. Coupling members having widths of 0,64 1,3 and 1,9 mm (0.025, 0.050 and 0.075 inches) have been applied to the illustrated stent member having a peak-to-peak undulation amplitude of about 1,9 mm (0.075 inch) with suitable results. However, it has been found that as the coupling member band width increases, the stent-graft flexibility generally is diminished. It is believed that a coupling member width of about one-fourth to three-fourths the amplitude of undulations **14,** measured peak-to-peak, is preferred (more preferably about one-third to two-thirds that amplitude) to optimize flexibility. It also has been found that by positioning one of lateral margins of the ribbon-shaped coupling member 8 adjacent to the apexes, e.g., in abutment with linking member **20,** the coupling member width may be reduced without significantly sacrificing apex securement. Varying the width of the coupling member can also result in the adjustment of other structural properties. Increasing the width can also potentially increase the radial stiffness and the burst pressure and decrease the porosity of the device. Increasing band width can also diminish graft member wrinkling between coupling member turns.

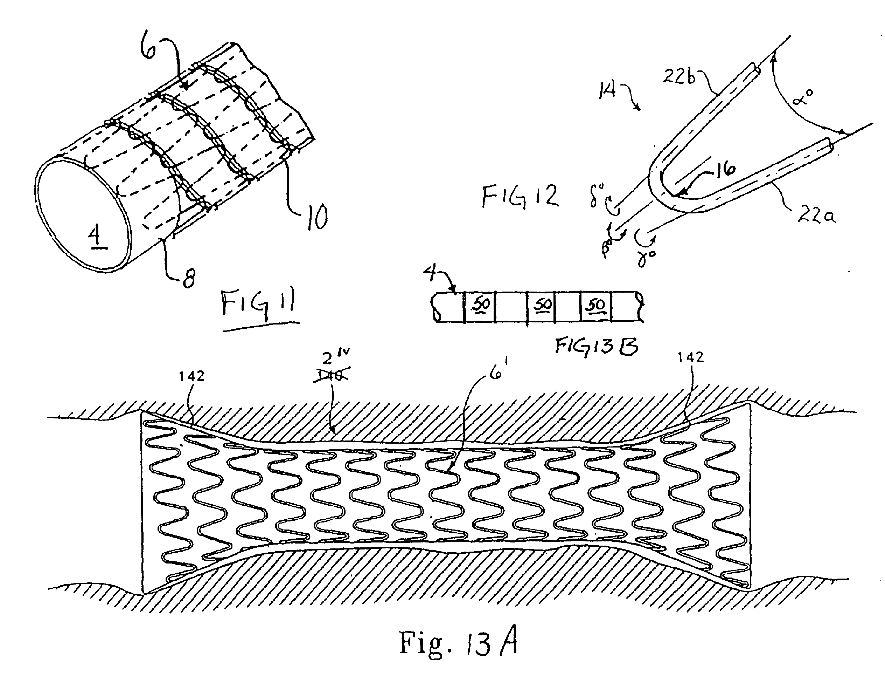

Coupling member **8** (or separate pieces thereof) also surrounds the terminal end portions of the stent-graft to secure the terminal portions of the graft member to the support structure formed by stent member **6** as shown in Fig. 11, for example.

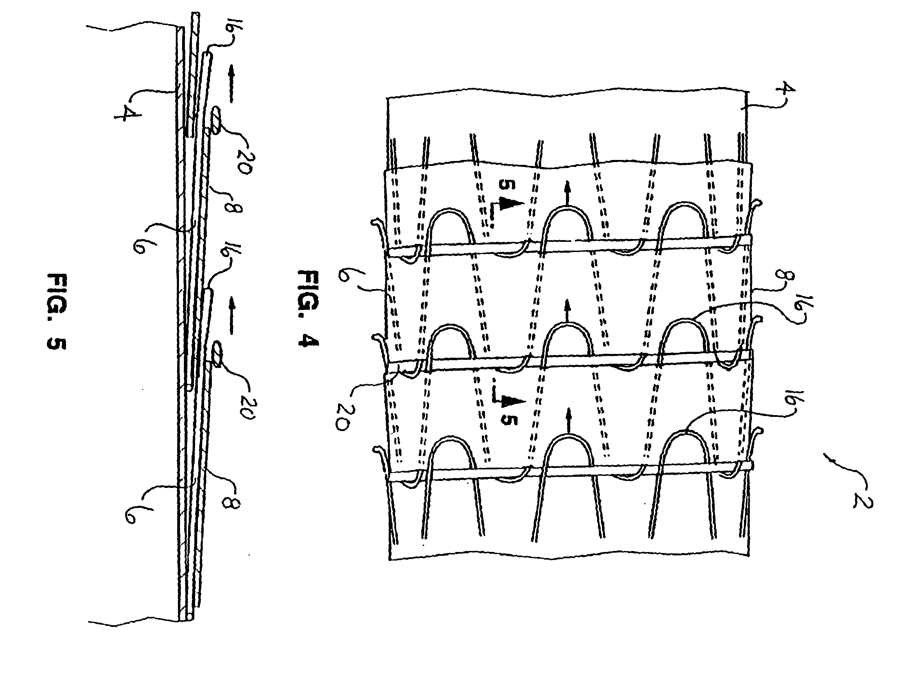

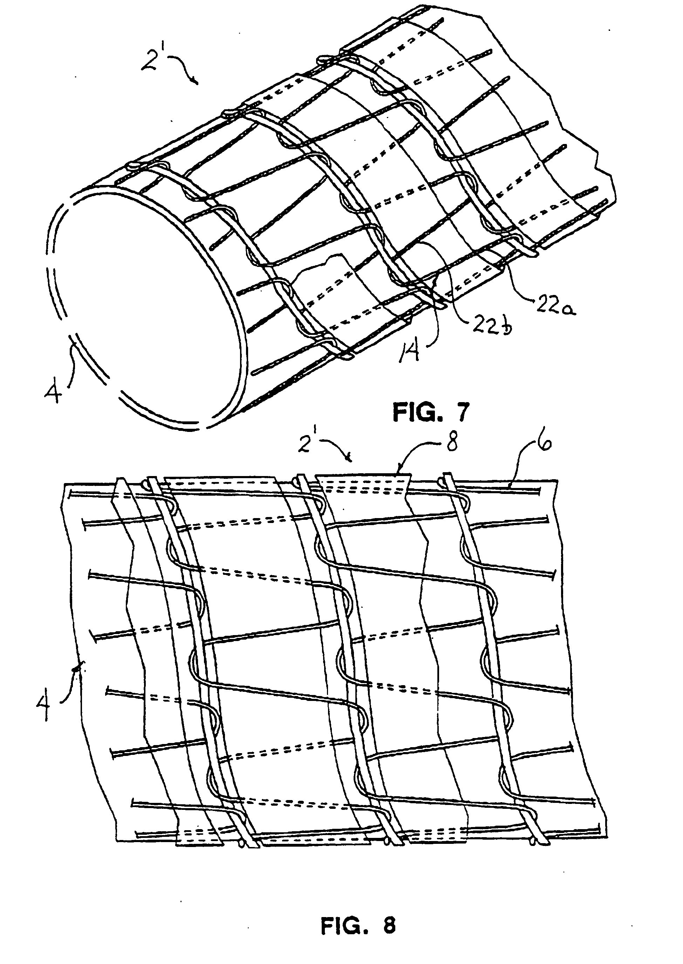

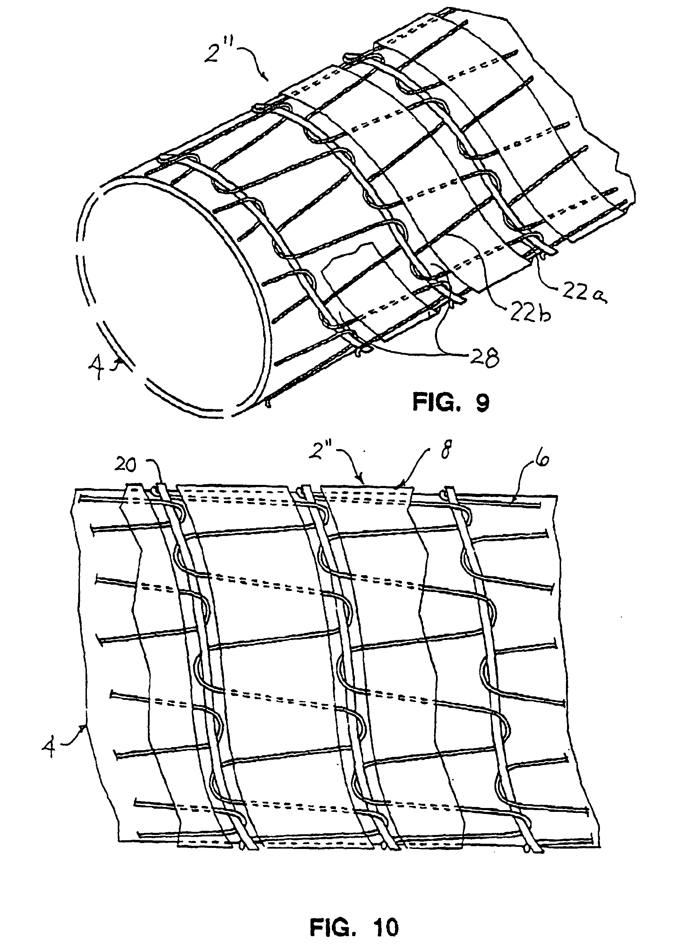

Although the coupling member may cover a substantial portion of each undulation as discussed above, apex portions **16** may still move within the undulations in-phase therewith as shown in Figs. 4-6 due primarily to the flexibility of coupling and linking members **8** and **20,** respectively. Further, coupling member **8** may be wrapped so as to be completely external to stent member **6** as shown in Figs. 1-6, interwoven above and below alternating undulations **14** as shown in Figs. 7 and 8 or interwoven above and below alternating undulation arms **22a** and **22b** as shown in Figs. 9 and 10. In addition, the ribbon-shaped tape or coupling member **8** may be axially spaced away from the apexes and linking member **20** (Figs. 9 and 10) as compared to the embodiments shown in Figs. 1-8. This spacing provides an area **28** in which linking member **20** can freely move without restraint, thereby reducing any resistance placed on apexes moving into corresponding undulations during compression or bending.

Although a particular coupling member configuration and pattern has been illustrated and described, other configurations and/or patterns may be used without departing from the scope of the present invention. For example, coupling member(s) arranged in a multiple helix (e.g., a double or triple helix) may be used. Longitudinally extending strips of ribbon may be used and may be preferred when the coupling member is used in conjunction with other stent member configurations.

Each undulation **14** alternatively may be described as a torsion segment and for purposes of the following discussion will be referred to as a torsion segment **14.** Referring to Fig. 12, an isolated undulation **14** is shown to facilitate the following discussion involving stent mechanics involved in deployment of the device. Each torsion segment includes an apex portion 16 and two adjacent torsion arms or lengths **22a** and **22b** extending therefrom. Typically, then, each torsion arm **22a & b** will be a component of each of two adjacent torsion segments **14.** When torsion segment **14** undergoes a flexing in the amount of α°, apex portion **16** will flex some amount β°, torsion arm **22a** will undertake a twist of γ°, and torsion arm **22b** will undertake a twist opposite of that found in torsion arm **22a** in the amount of δ°. The amounts of angular torsion found in the torsion arms **(22a** & **22b)** will not necessarily be equal because the torsion arms are not necessarily at the same angle to the longitudinal axis of the stent-member. Nevertheless, the sum of β°+γ°+δ° will equal α°. When a value of α° is chosen, as by selection of the shape and size of the stent-member upon folding, the values of the other three angles ( β°+γ°+δ°) are chosen by virtue of selection of number of torsion segments around the stent, size and physical characteristics of the wire, and length of the torsion arms **(22a & b).** Each of the noted angles must not be so large as to exceed the values at which the chosen material of construction plastically deforms at the chosen value of α°.

To further explain: it should be understood that torsion segment **14** undergoes a significant amount of flexing as the stent-member is folded or compressed in some fashion. The flexing provides a twist to the torsion arms **(22a & b),** a significant portion of which is generally parallel to the longitudinal axis of the stent.

The described stent-member uses concepts which can be thought of as widely distributing and storing the force necessary to fold the tubular stent into a configuration which will fit through a diameter smaller than its relaxed outside diameter without inducing plastic deformation of the constituent metal or plastic and yet allowing those distributed forces to expand the stent upon deployment.

Once the concept of distributing the folding or compression stresses both into a bending component (as typified by angle β° in Fig. 12) and to twisting components (as typified by angle γ° and δ° in Fig. 12), and determining the overall size of a desired stent, determination of the optimum materials as well as the sizes of the various integral components making up the stent becomes straightforward. Specifically, the diameter and length of torsion lengths, apex portion dimensions and the number of torsion segments around the stent may then be determined.

Referring to Fig. 13A, a stent-graft **2^{iv}** differing from stent-graft **2** in graft support structure is shown. Stent-graft **2^{iv}** includes stent member **6',** which is the same as stent member **6** with the exception that it includes flared end portions **142** at one or both ends. Flared end portions **142** provide secure anchoring of the resulting stent-graft **2^{iv}** against the vessel wall and prevents the implant from migrating downstream. In addition, flared end portions **142** provide a tight seal against the vessel so that the blood is channeled through the lumen rather than outside the graft. The undulating structure may vary in spacing to allow the helical turns to maintain their phased relationship as discussed above. Although a linking member between the contiguous helical turns is not shown, such structure preferably is included to maintain the alignment of the apexes as discussed above.

The graft support structure also may be made by forming a desired structural pattern out of a flat sheet. The sheet may then be rolled to form a tube. The stent also may be machined from tubing. If the chosen material is nitinol, careful control of temperature during the machining step may be had by EDM (electro-discharge-machining), laser cutting, chemical machining, or high pressure water cutting. As shown in Fig. 13B, the stent-member (or graft support structure) may comprise multiple tubular members or sections 50, each coupled to the graft-member 4 with a coupling member as described above. Tubular members or sections 50 may be configured to have the same construction as stent-member 4 shown in Figs. 1-11, for example. However, other stent constructions may be used. Tubular members **50** also may be directly coupled to each other (e.g., with bridging element(s) extending between adjacent sections as would be apparent to one of ordinary skill), or indirectly coupled to each other through their interconnection with the graft member.

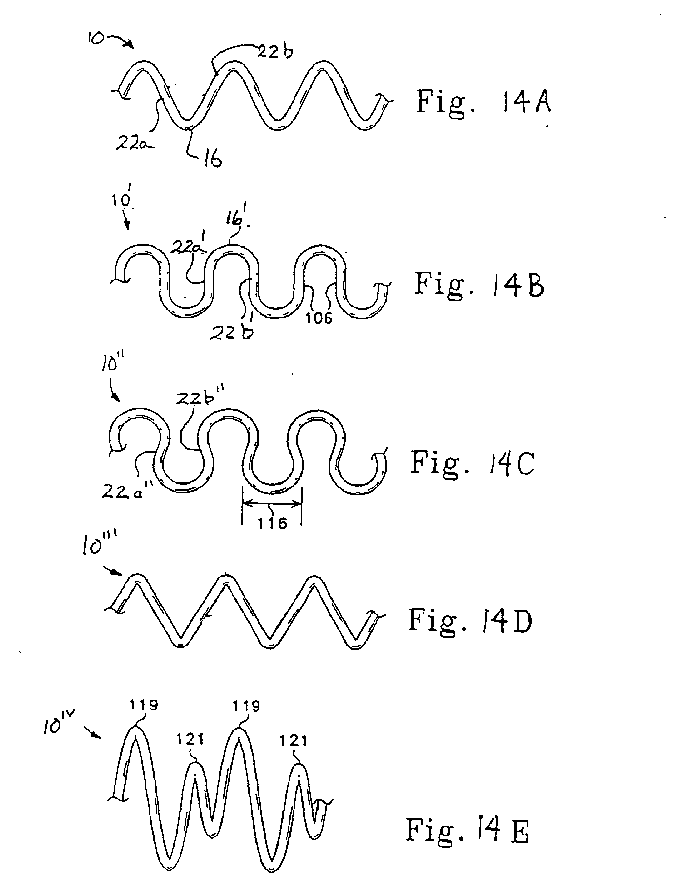

Referring to Figs. 14A-E, various undulation configurations suitable for the present invention are shown. Fig. 14A shows the sinusoidal shaped undulating member **10** described above. Adjacent torsion arms **22a & b** are not parallel. Fig. 14B shows an undulating member **10'** having generally U-shaped undulations or torsion members where the torsion arms are generally parallel. Fig. 14C shows a further variation where undulating member **10"** includes ovoid shaped undulations or torsion segments. In this variation, adjacent torsion arms **22"a & b** are again not parallel, but generally form an open-ended oval. Fig. 14D shows another variation where undulating member **10'"** includes V-shaped torsion members. In this variation, the adjacent torsion arms **120** form a relatively sharp angle at the respective apex portions. Fig. 14E shows undulating member **10^{iv}** in which adjacent undulations have different amplitudes. The peaks of the large amplitude torsion segments **119** may be lined up "out of phase" or "peak to peak" with small or large amplitude torsion segments **119, 121,** respectively, in the adjacent turn of the helix or may be positioned "in phase" similar to those discussed with regard to Figs. 1A and B above. The configurations shown in Figs. 14A-14E are exceptionally kink-resistant and flexible when flexed along the longitudinal axis of the stent-member.

As discussed above, the stent member preferably is oriented coaxially with the tubular graft member. Although the stent member may be placed within the graft member, it preferably is placed on the outer surface of the graft member so that a relatively smooth graft wall interfaces with the blood. In certain configurations, an additional graft member may be placed on the outer surface of the stent-graft illustrated in the drawings. When the multiple graft structure is utilized, the stent structure should have the strength and flexibility to urge the graft tubing firmly against the vessel wall so that the graft member conforms with the inner surface of the vessel wall. In addition, the graft member preferably is impermeable to blood at normal or physiologic blood pressures. The impermeability makes the stent-graft suitable for shunting and thereby hydraulically isolating aneurysms.

The scope of materials suitable for the stent and graft members and the linking member as well as deployment mechanisms will be discussed in detail below.

### Stent Materials

The stent member is constructed of a reasonably high strength material, i.e., one which is resistant to plastic deformation when stressed. Preferably, the stent member comprises a wire which is helically wound around a mandrel having pins arranged thereon so that the helical turns and undulations can be formed simultaneously. Other constructions also may be used. For example, an appropriate shape may be formed from a flat stock and wound into a cylinder or a length of tubing formed into an appropriate shape.

In order to minimize the wall thickness of the stent-graft, the stent material should have a high strength-to-volume ratio. Use of designs as depicted herein provides stents which may be longer in length than conventional designs. Additionally, the designs do not suffer from a tendency to twist (or helically unwind) or to shorten as the stent-graft is deployed. As will be discussed below, materials suitable in these stents and meeting these criteria include various metals and some polymers.

A percutaneously delivered stent-graft must expand from a reduced diameter, necessary for delivery, to a larger deployed diameter. The diameters of these devices obviously vary with the size of the body lumen into which they are placed. For instance, the stents of this invention may range in size from 2.0mm in diameter (for neurological applications) to 40mm in diameter (for placement in the aorta). A range of about 2.0mm to 6.5mm (perhaps to 10.0mm) is believed to be desirable. Typically, expansion ratios of 2:1 or more are required. These stents are capable of expansion ratios of up to 5:1 for larger diameter stents. Typical expansion ratios for use with the stents-grafts of the invention typically are in the range of about 2:1 to about 4-1 although the invention is not so limited. The thickness of the stent materials obviously varies with the size (or diameter) of the stent and the ultimate required yield strength of the folded stent. These values are further dependent upon the selected materials of construction. Wire used in these variations are typical of stronger alloys, e.g., nitinol and stronger spring stainless steel, and have diameters of about 0.05 to 0,1 mm (0.002 inches to 0.005 inches). For the larger stents, the appropriate diameter for the stent wire may somewhat larger, e.g., 0,1 to 0,51 mm (0.005 to 0.020 inches). For flat stock metallic stents, thicknesses of about 0.05 to 0.1 mm (0.002 inches to 0.005 inches) is usually sufficient. For the larger stents, the appropriate thickness for the stent flat stock may be somewhat thicker, e.g., 0.1 to 0,51 mm (0.005) to 0.020 inches).

The stent-graft is fabricated in the expanded configuration. In order to reduce its diameter for delivery the stent-graft would be folded along its length, similar to the way in which a ballon would be folded. It is desirable, when using super elastic alloys which are also have temperature memory characteristics, to reduce the diameter of the stent at a temperature below the transition-temperature of the alloys. Often the phase of the alloy at the lower temperature is somewhat more workable and easily formed. The temperature of deployment is desirably above the transition temperature to allow use of the super-elastic properties of the alloy.

There are a variety of disclosures in which super-elastic alloys such as nitinol are used in stents. See. U.S. Patent Nos. 4,503,569, to Dotter; 4,512,338, to Balko et al.; 4,990,155, to Wilkoff; 5,037,427, to Harada. et al.; 5,147,370, to MacNamara et al.; 5,211,658, to Clouse; and 5,221,261, to Termin et al. None of these references suggest a device having discrete, individual, energy-storing torsional members.

Jervis, in U.S. Pat. Nos. 4,665,906 and 5,067,957, describes the use of shape memory alloys having stress-induced martensite properties in medical devices which are implantable or, at least, introduced into the human body.

It should be clear that a variety of materials variously metallic, super elastic alloys, and preferably nitinol, are suitable for use in these stents. Primary requirements of the materials are that they be suitably springy even when fashioned into very thin sheets or small diameter wires. Various stainless steels which have been physically, chemically, and otherwise treated to produce high springiness are suitable as are other metal alloys such as cobalt chrome alloys (e.g., ELGILOY®), platinum/tungsten alloys, and especially the nickel-titanium alloys generically known as "nitinol".

Nitinol is especially preferred because of its "super-elastic" or "pseudoelastic" shape recovery properties, i.e., the ability to withstand a significant amount of bending and flexing and yet return to its original form without deformation. These metals are characterized by their ability to be transformed from an austenitic crystal structure to a stress-induced martensitic structure at certain temperatures, and to return elastically to the austenitic shape when the stress is released. These alternating crystalline structures provide the alloy with its super-elastic properties. These alloys are well known but are described in U.S. Pat. Nos. 3,174,851, 3,351,463, and 3,753,700. Typically, nitinol will be nominally 50.6% (±0.2%) Ni with the remainder Ti. Commercially available nitinol materials usually will be sequentially mixed, cast, formed, and separately cold-worked to 30-40%, annealed, and stretched. Nominal ultimate yield strength values for commercial nitinol are in the range of 30 psi and for Young's modulus are about 700 Kbar. The '700 patent describes an alloy containing a higher iron content and consequently has a higher modulus than the Ni-Ti alloys.

Nitinol is further suitable because it has a relatively high strength to volume ratio. This allows the torsion members to be shorter than for less elastic metals. The flexibility of the stent-graft is largely dictated by the length of the torsion segments and/or torsion arms. The shorter the pitch of the device, the more flexible the stent-graft structure can be made. Materials other than nitinol are suitable. Spring tempered stainless steels and cobalt-chromium alloys such as ELGILOY® are also suitable as are a wide variety of other known "super-elastic" alloys.

Although nitinol is preferred in this service because of its physical properties and its significant history in implantable medical devices, we also consider it also to be useful in a stent because of its overall suitability with magnetic resonance imaging (MRI) technology. Many other alloys, particularly those based on iron, are an anathema to the practice of MRI causing exceptionally poor images in the region of the alloy implant. Nitinol does not cause such problems.

Other materials suitable as the stent include certain polymeric materials, particularly engineering plastics such as thermotropic liquid crystal polymers ("LCP's"). These polymers are high molecular weight materials which can exist in a so-called "liquid crystalline state" where the material has some of the properties of a liquid (in that it can flow) but retains the long range molecular order of a crystal. The term "thermotropic" refers to the class of LCP's which are formed by temperature adjustment. LCP's may be prepared from monomers such as p,p'-dihydroxy-polynuclear-aromatics or dicarboxy-polynuclear-aromatics. The LCP's are easily formed and retain the necessary interpolymer attraction at room temperature to act as high strength plastic artifacts as are needed as a foldable stent. They are particularly suitable when augmented or filled with fibers such as those of the metals or alloys discussed below. It is to be noted that the fibers need not be linear but may have some preforming such as corrugations which add to the physical torsion enhancing abilities of the composite.

### Linking Member Materials

Flexible link 20, which is slidably disposed between adjacent turns of the helix may be of any appropriate filamentary material which is blood compatible or biocompatible and sufficiently flexible to allow the stent to flex and not deform the stent upon folding. Although the linkage may be a single or multiple strand wire (platinum, platinum/tungsten, gold, palladium, tantalum, stainless steel, etc.), much preferred in this invention is the use of polymeric biocompatible filaments. Synthetic polymers such as polyethylene, polypropylene, polyurethane, polyglycolic acid, polyesters, polyamides, their mixtures, blends, copolymers, mixtures, blends and copolymers are suitable; preferred of this class are polyesters such as polyethylene terephthalate including DACRON® and MYLAR® and polyaramids such as KEVLAR®, polyfluorocarbons such as polytetrafluoroethylene with and without copolymerized hexafluoropropylene (TEFLON® or GORE-TEX®), and porous or nonporous polyurethanes. Natural materials or materials based on natural sources such as collagen may also be used is this service.

### Graft Member Materials

The tubular component or graft member of the stent-graft may be made up of any material which is suitable for use as a graft in the chosen body lumen. Many graft materials are known, particularly known are those used as vascular graft materials. For instance, natural materials such as collagen may be introduced onto the inner surface of the stent and fastened into place. Desirable collagen-based materials include those described in U.S. Pat. No. 5,162,430, to Rhee et al, and WO 94/01483 (PCT/US93/06292). Synthetic polymers such as polyethylene, polypropylene, polyurethane, polyglycolic acid, polyesters, polyamides, their mixtures, blends, copolymers, mixtures, blends and copolymers are suitable; preferred of this class are polyesters such as polyethylene terephthalate including DACRON® and MYLAR® and polyaramids such as KEVLAR®, polyfluorocarbons such as polytetrafluoroethylene (PTFE) with and without copolymerized hexafluoropropylene (TEFLON® or GORE-TEX®), and porous or nonporous polyurethanes. Especially preferred in this invention are the expanded fluorocarbon polymers (especially PTFE) materials described in British. Pat. Nos. 1, 355, 373, 1,506, 432, or 1, 506, 432 or in U.S. Pat. Nos. 3,953,556, 4,187,390, or 5,276,276.

Included in the class of preferred fluoropolymers are polytetrafluoroethylene (PTFE), fluorinated ethylene propylene (FEP), copolymers of tetrafluoroethylene (IFE) and per fluoro(propyl vinyl ether) (PFA), homopolymers of polychlorotrifluoroethylene (PCTFE), and its copolymers with TFE, ethylene-chlorotrifluoroethylene (ECTFE), copolymers of ethylene-tetrafluoroethylene (ETFE), polyvinylidene fluoride (PVDF), and polyvinyfluoride (PVF). Especially preferred, because of its widespread use in vascular prostheses, is expanded PTFE.

In addition, one or more radio-opaque metallic fibers, such as gold, platinum, platinum-tungsten, palladium, platinum-iridium, rhodium, tantalum, or alloys or composites of these metals like may be incorporated into the device, particularly, into the graft, to allow fluoroscopic visualization of the device.

The tubular component may also be reinforced using a network of small diameter fibers. The fibers may be random, braided, knitted, or woven. The fibers may be imbedded in the tubular component, may be placed in a separate layer coaxial with the tubular component, or may be used in a combination of the two.

A preferred material for the graft and coupling members is porous expanded polytetrafluorethylene. An FEP coating is one preferred adhesive that is provided on one side of the coupling member.

### Manufacture of the Stent-Graft:

The following example is provided for purposes of illustrating a preferred method of manufacturing a stent-graft constructed according to the present invention which in this example case is the stent-graft shown in Figs. 1-6. It should he noted, however, that this example is not intended to limit the invention.

The stent member wire is helically wound around a mandrel having pins positioned thereon so that the helical structure and undulations can be formed simultaneously. While still on the mandrel, the stem member is heated to about 460°F for about 20 minutes so that it retains its shape.

Wire sizes and materials may vary widely depending on the application. The following is an example construction for a stent-graft designed to accommodate a 6mm diameter vessel lumen. The stent member comprises a nitinol wire (50.8 atomic % Ni) having a diameter of about 0.2 mm (0.007 inch). In this example case, the wire is formed to have sinusoidal undulations, each having an amplitude measured peat-to-peak of about 2.54 mm (0.100 inch) and the helix is formed with a pitch of about 10 windings per 25.4 mm (inch). The inner diameter of the helix (when unconstrained) is about 6.8mm. The linking member can be arranged as shown in the drawings and may have a diameter of about 0,2 mm (0.006 inch).

In this example, the graft member is parous expanded polytetrafluorethylene (PTFE), while the coupling member is expanded PTFE coated with FEP. The coupling member is in the form of a flat ribbon (as shown in the illustrative embodiments) that is positioned around the stent and graft members as shown in Figs. 1-3. The side of the coupling member or ribbon that is FEP coated faces the graft member to secure it to the graft member. The intermediate stent-graft construction is heated to allow the materials of the ribbon and graft member to merge and self-bind as will be described in more detail below.

The FEP-coated porous expanded PTFE film used to form the ribbon shaped coupling member preferably is made by a process which comprises the steps of:
(a) contacting a porous PTFE film with another layer which is preferably a film of FEP or alternatively of another thermoplastic polymer;
(b) heating the composition obtained in step (a) to a temperature above the melting point of the thermoplastic polymer;
(c) stretching the heated composition of step (b) while maintaining the temperature above the melting point of the thermoplastic polymer; and
(d) cooling the product of step (c).

In addition to FEP, other thermoplastic polymers including thermoplastic fluoropolymers may also be used to make this coated film. The adhesive coating on the porous expanded PTFE film may be either continuous (non-porous) or discontinuous (porous) depending primarily on the amount and rate of stretching, the temperature during stretching, and the thickness of the adhesive prior to stretching.

The thin wall expanded PTFE graft used to construct this example was of about .1mm (0.004 in) thickness and had a density of about .5g/cc. The microstructure of the porous expanded PTFE contained fibrils of about 25 micron length. A 3cm length of this graft material was placed on a mandrel the same diameter as the inner diameter of the graft. The nitinol stent member having about a 3cm length was then carefully fitted over the center of the thin wall graft.

The stent-member was then provided with a ribbon shaped coupling member comprised of the FEP coated film as described above. The coupling member was helically wrapped around the exterior surface of the stent-member as shown in Figs. 1-6. The ribbon shaped coupling member was oriented so that its FEP-coated side faced inward and contacted the exterior surface of the stent-member. This ribbon surface was exposed to the outward facing surface of the thin wall graft member exposed through the openings in the stent member. The uniaxially-oriented fibrils of the microstructure of the helically-wrapped ribbon were helically-oriented about the exterior stent surface.

The mandrel assembly was placed into an oven set at 315°C for a period of 15 minutes after which the film-wrapped mandrel was removed from the oven and allowed to cool. Following cooling to approximately ambient temperature, the mandrel was removed from the resultant stent-graft. The amount of heat applied was adequate to melt the FEP-coating on the porous expanded PTFE film and thereby cause the graft and coupling members to adhere to each other. Thus, the graft member was adhesively bonded to the inner surface of helically-wrapped coupling member 8 through the openings between the adjacent wires of the stent member. The combined thickness of the luminal and exterior coverings (graft and coupling members) and the stent member was about 0.4mm.

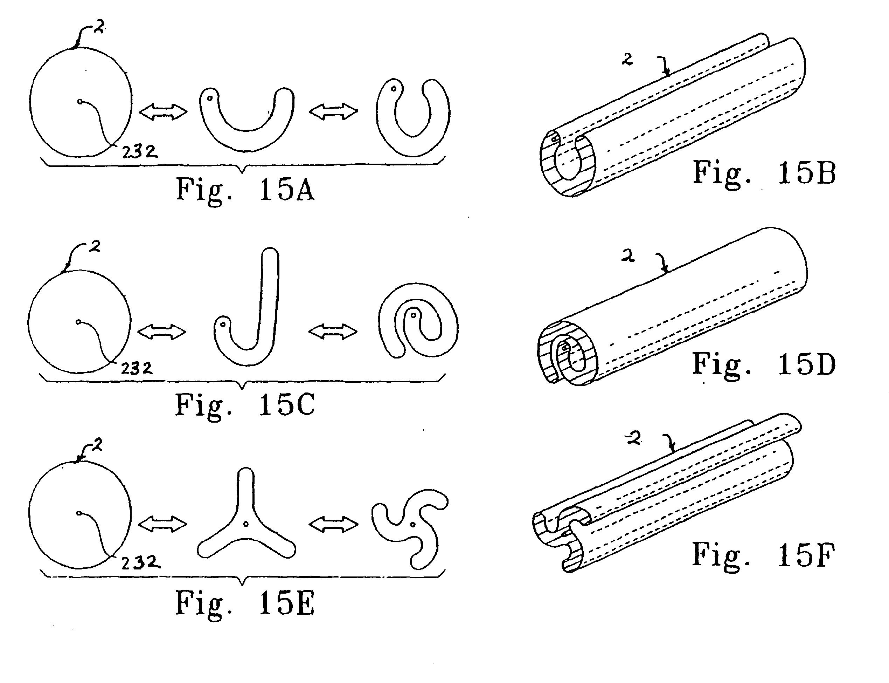

The stent-graft was then folded in order to prepare it for delivery. To accomplish this a stainless steel wire which was a couple of inches longer than the stent-graft was inserted through the lumen of the stent-graft. The stent-graft was flattened and the stainless steel wire positioned at one end of the stent-graft. A second stainless sire of about the same length was place on the outer surface of the stent-graft adjacent to the first stainless steel wire. The wires were then mounted together into a fixture, tensioned and then rotated, thereby folding the stent-graft as shown in Figs. 15 C & D which will be discussed in more detail below. As the stent-graft rotates it is pressed into a "C" shaped elongated stainless steel clip in order to force it to roll upon itself. The folded stent-graft is then advanced along the wire out of the clip into a glass capture tube. A removable tether line, which is used to constrain the stent-graft in the rolled configuration for delivery, as will be discussed in more detail below, is applied to the stent-graft at this point by gradually advancing the stent-graft out of the capture tube and lacing the tether line through the stent-graft structure. After this step is completed, the stent-graft is pulled off of the first wire and transferred onto the distal end of the catheter shaft or tubing for delivery.

Prior to folding, the stent-graft was cooled to about -30°C so that the nitinol was fully martensitic and, thus, malleable. This is done to allow the stent-graft to be more easily folded. Cooling is accomplished by spray soaking the graft with chilled gas such as tetrafluroethane. Micro-Dust (dry circuit duster manufactured by MicroCare Corporation (Conn) provides suitable results. The spray canister was held upside down to discharge the fluid as a liquid onto the stent-graft.

### Deployment of the Stent-Graft

The stent-graft may be delivered percutaneously, typically through the vasculature, after having been folded to a reduced diameter. Once reaching the intended delivery site, it may be expanded to form a lining on the vessel wall.

When a stent-graft having torsion members, as described above, is folded, crushed, or otherwise collapsed, mechanical energy is stored in torsion in those members. In this loaded state, the torsion members have a torque exerted about them and consequently have a tendency to untwist. Collectively, the torque exerted by the torsion members as folded down to a reduced diameter must be restrained from springing open. The stent-member preferably has at least one torsion member per fold. The stent-graft is folded along its longitudinal axis and restrained from springing open. The stent-graft is then deployed by removing the restraining mechanism, thus allowing the torsion members to spring open against the vessel wall. The attending physician will select an appropriately sized stent-graft. Typically, the stent-graft will be selected to have an expanded diameter of up to about 10% greater than the diameter of the lumen at the deployment site. Although the stent-graft may have other constructions as discussed above, the following deployment examples are made with reference to stent-graft **2.**

Fig. 15A diagrammatically illustrates a folding sequence for folding a stent-graft constructed according to the present invention. The stent-graft, generally designated with reference numeral **2** is positioned about a guidewire **232** and folded into a loose C-shaped configuration. Fig. 15B shows a diagrammatic perspective view of the resulting folded stent-graft. Figs. 15C & E show further folding sequences. Figs. 15D & F show diagrammatic perspective views of the resulting folded stent-grafts showing the rolled and triple lobed configurations, respectively. The rolled configuration is preferred.

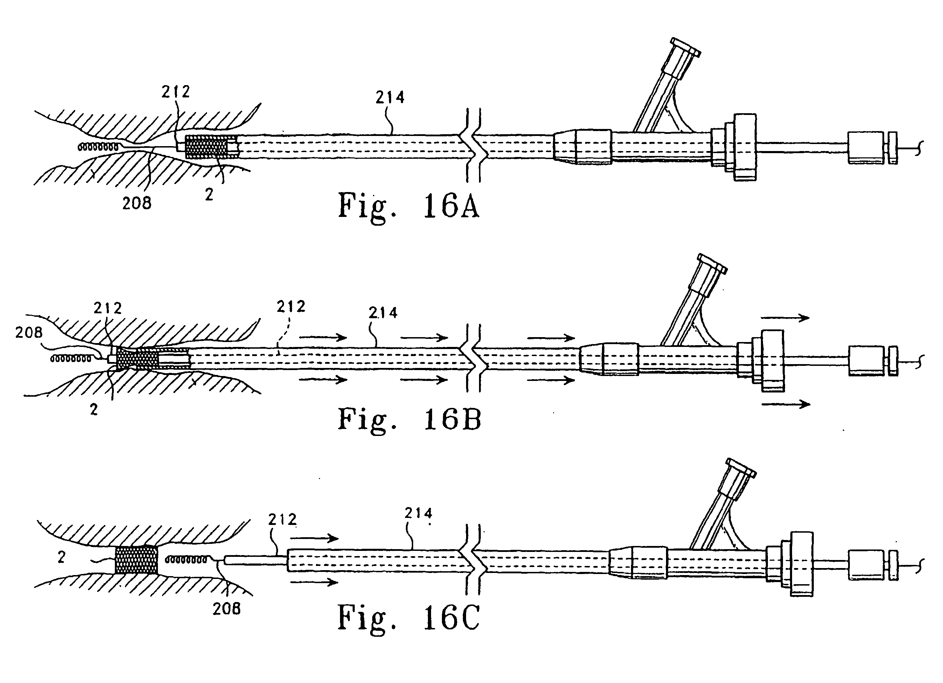

Figs. 16A-16C diagrammatically illustrate one method of deploying the present invention. Fig. 16A shows an example target site having a narrowed vessel lumen. A guidewire **208** having a guide tip has been directed to the site using known techniques. The stent-graft, e.g., stent-graft **2** is mounted on guidewire tubing **212** inside outer sliding sheath **214** after having been folded in the manner discussed above. The outer sliding sheath **214** binds the compressed stent-graft **2** in place until released.

Fig. 16B shows placement of the stent-graft **2** at the selected site by sliding the stent-graft over the guidewire all together with the guidewire tubing **212** and the outer sliding sheath **214.** The stent-graft is deployed by holding the guidewire tubing **212** in a stationary position while withdrawing the outer sliding sheath **214.** Fig. 16B shows the stent-graft partially deployed, while Fig. 16C shows the stent-graft fully deployed after the guidewire tubing and the outer sliding sheath have been fully retracted.

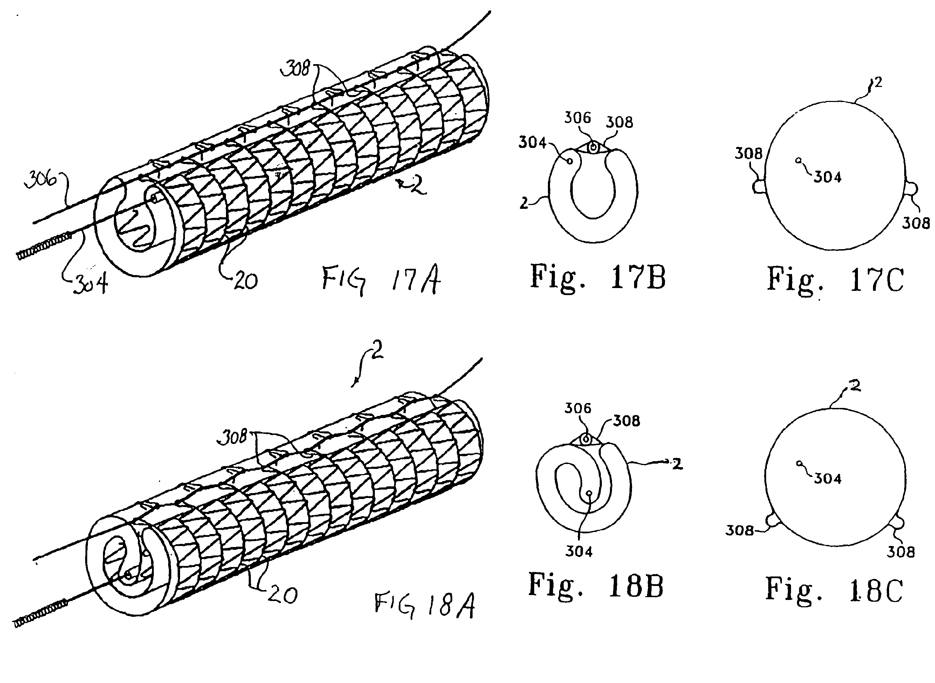

Figs. 17A-C, 18A-C, and 19A-C show deployment variations for deploying a stent-graft constructed according to the present invention. These methods involve the use of a control line or tether line which maintains the stent or stent-graft in a folded configuration until release. Referring to Figs. 17A & B, diagrammatically represented stent-graft 2 is shown folded about guidewire **304** so that, when deployed, the guidewire **304** is within stent-graft 2. A tether wire **306** is passed through loops **308** which preferably are formed by pulling the linking member discussed above away from the stent structure. When tether wire **306** is removed by sliding it axially along the stent-graft and out of loops **308,** the stent-graft unfolds into a generally cylindrical shape (Fig. 17C). Referring to Figs. 18A & B stent-graft **2** is shown in a rolled pre-deployment configuration. In this case, guidewire **304** is inside the stent. When expanded by removal of tether wire **306,** the stent-graft assumes the form shown in Fig. 18C.

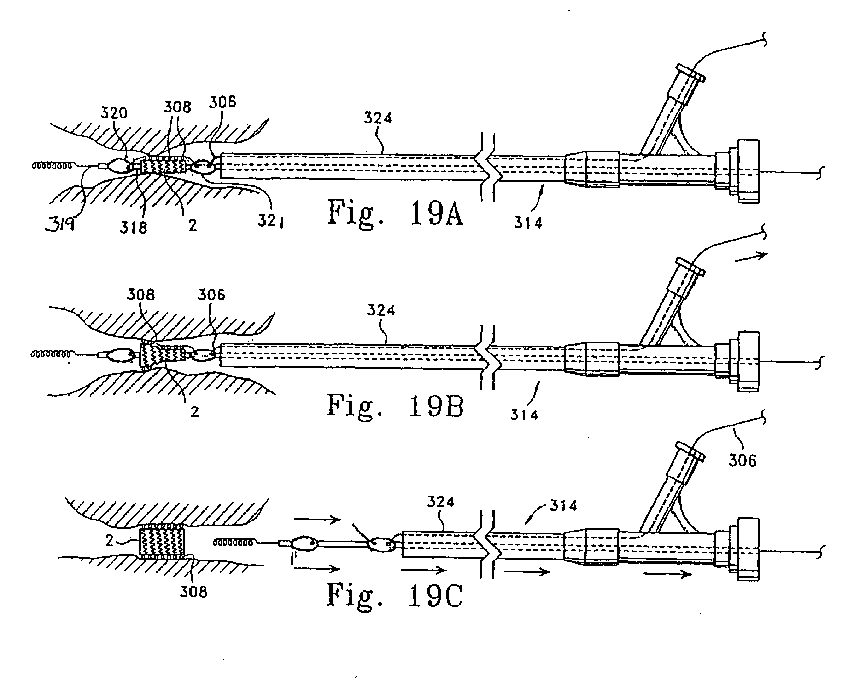

Figs. 19A-C diagrammatically show additional procedures for deploying a stent-graft of the present invention using a percutaneous catheter assembly **314.** Referring to Fig. 19A, catheter assembly **314** has been inserted to a selected site within a body lumen. A stent-graft such as stent-graft **2** is folded about guidewire **319** and guidewire tube **318** held axially in place prior to deployment by distal barrier **320** and proximal barrier **321.** The distal and proximal barriers typically are affixed to the guidewire tube **318.** Tether wire **306** extends through loops **308** proximally through the catheter assembly's **314** outer jacket **324** through to outside the body. Tether wire **306** may be outside proximal barrier **321** or extend therethrough as shown in Fig. 19A. Fig. 19B shows partial removal of tether wire **306** from loops **308** to partially expand the stent-graft **312** onto the selected site. Fig. 19C shows complete removal of the tether wire, the loops and retraction of the catheter assembly **314** from the interior of the stent-graft which is fully expanded.

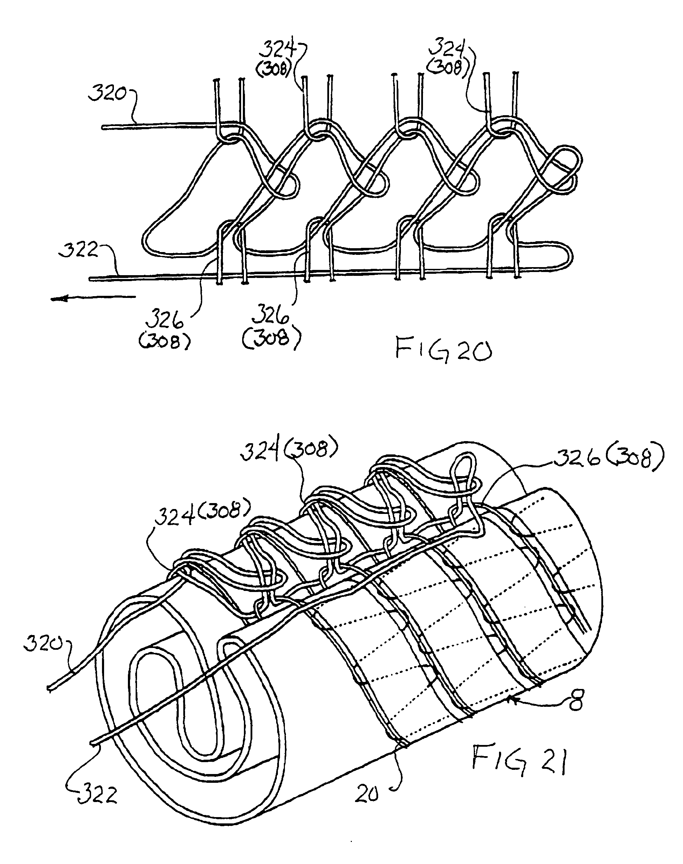

Fig. 20 shows an enlarged view of a stent fold line having the familiar herringbone pattern of the preferred "sack knot" used to close the fold in the stent. This knot is the one used to hold, e.g., burlap sacks of feed grain closed prior to use and yet allow ease of opening when the sack is to be opened. In this variation, the slip line has a fixed end **320** and a release end **322.** Loops of the slip line pass through the eyelets **324** on the side of the stent fold associated with the fixed end **320** and are held in place by eyelets **326** on the side of the stent fold associated with the release end **322.** The fixed end **320** is not typically tied to the stent so to allow removal of the slip line after deployment. The eyelets **324** and **326** are desirable but optional. The eyelets **324** and **326** may be wire or polymeric thread or the like tied to the stent structure at the edge of the stent fold. Alternatively, eyelets **324** and **326** may be formed from linking member **20** as discussed above to form the loops designated with reference numeral **308.** In a further alternative, the slip line may be woven into the stent structure, e.g., into undulations **14.** The self-expanding stent may be deployed by pulling axially on release end **322** as shown by the arrow in the drawing. When the release end **322** is in the vicinity of the proximal end of the stent-graft (i.e., the end closest to the hub adapter when delivered, for example, through catheter assembly **314),** the stent-graft unfolds from the distal to proximal end as shown in Figs. 16B and 19B, for example.

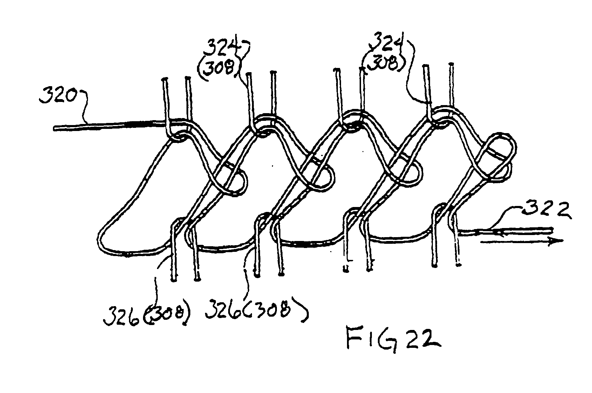

Fig. 21 is a diagrammatic perspective view of a folded stent-graft using the knot shown in Fig. 20. Fig. 21 shows the use of multiple stent folds similar in configuration to those described above. As was shown in Fig. 20, the fixed end portion **320** of the slip line is associated with a row of eyelets **324** which preferably are formed by pulling local portions of linking member **20** away from the fold line, threading the slip line therethrough and then releasing the respective portion of the linking member. Alternatively, the eyelets may be tied or otherwise fixed to the stent. The release end **322** is associated with the other row of eyelets **326.**

Referring to Fig. 22, a variation on the sack knot shown in Figs. 20 and 21 is shown. In this arrangement, release end **322** also is positioned so that when the release mechanism is associated with the stent-graft, release end **322** is in the vicinity of the proximal portion of the stent-graft. Thus, when release end 322 is pulled, the stent-graft unfolds from the proximal to the distal end (i.e., opposite to that shown in Figs. 16B and 19B, for example). As shown in the drawings, this arrangement eliminates the extra folded-back length of the tether line leading to release end 322 and may reduce the likelihood of snagging between the tether or slip line and the stent member. This arrangement also may provide less fluid flow resistance when the stent-graft is deployed against the flow of blood which may improve positioning accuracy during deployment.

Although stent-graft deployment is described using a catheter for percutaneous delivery, it should be understood that other deployment techniques may be used. The folded stent-graft may also be deployed through artificial or natural body openings with a sheath or endoscopic delivery device, for example, and perhaps without a guidewire. Similarly, the stent-graft may be delivered manually during a surgical procedure.

The stent-graft of the present invention may be used, for example, to reinforce vascular irregularities and provide a smooth nonthrombogenic interior vascular surface for diseased areas in blood vessels, or to increase blood flow past a diseased area of a vessel by mechanically improving the interior surface of the vessel. The inventive stent-graft is especially suitable for use within smaller vessels between 2mm and 6mm in diameter but is equally suitable for significantly larger vessels. The inventive stent-graft may be self-expandable so that it may be percutaneously delivered in a folded state on an endovascular catheter or via surgical or other techniques and then expanded. The stent-graft construction described above also has an adjustable length. It is axially compressible. Generally, the portion(s) of the graft member not secured to the stent member may slightly wrinkle during compression. This length adjustability is especially advantageous during implantation procedures. It provides the physician the ability to adjust the length of the device in-vivo as required during the placement of the device.

It is generally difficult for a physician to accurately determine anatomical distances due to vessel tortuosity in different planes which often occurs in aorta/iliac aneurysmal disease. Also, it is important for the physician to accurately measure distances when placing an endovascular stent-graft so the entire aneurysmal length is covered, yet important vessel branches are not occluded. The stent-graft design of the present invention allows the physician to adjust its length during deployment allowing more accurate placement of the device.

The following example illustrates the steps involved in placing an adjustable variable-length stent-graft into a patient's anatomy. In this example, stent-graft 2 is referenced for illustrative purposes. Reference to this structure is not intended to limit the invention. The stent-graft generally is a single tubular design. In this example, it is placed into the thoracic aorta 70, and will be located between the renal arteries and the T-7 artery. The direction of deployment will be from renals upstream to the T-7 artery. The device will be supplied in its longest or extendible state with shortening capability during deployment (the inverse where a compressed stent-graft is deployed also is possible).

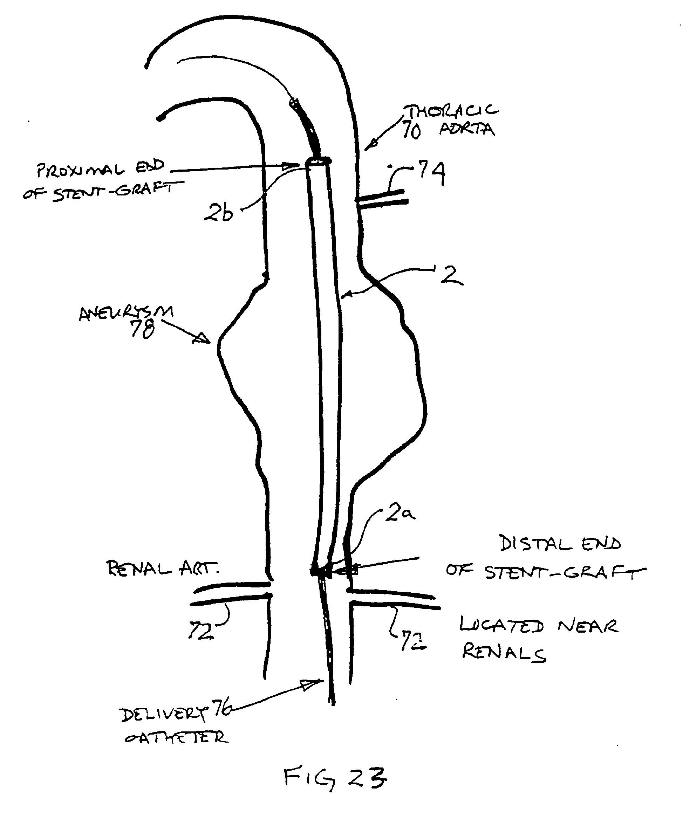

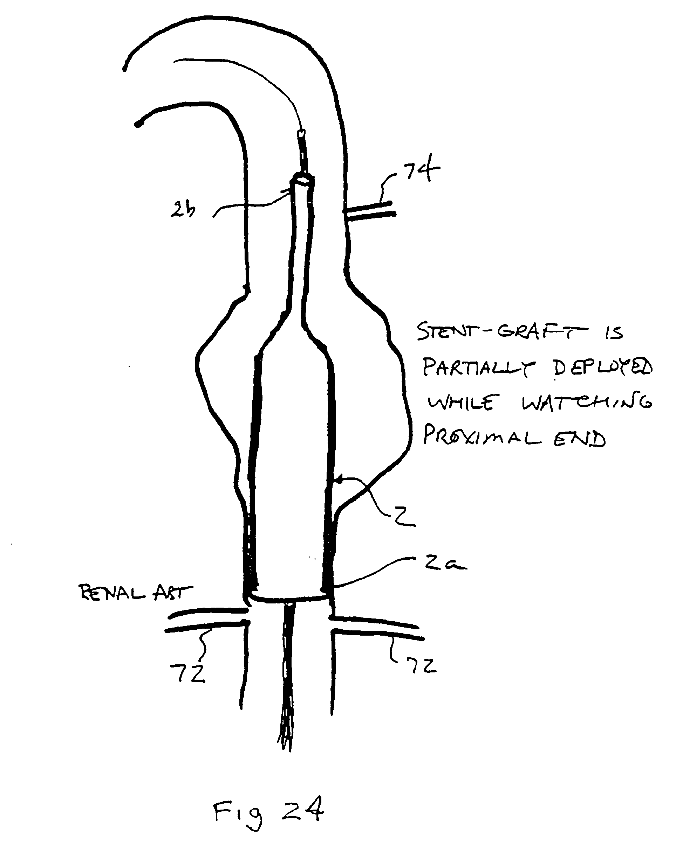

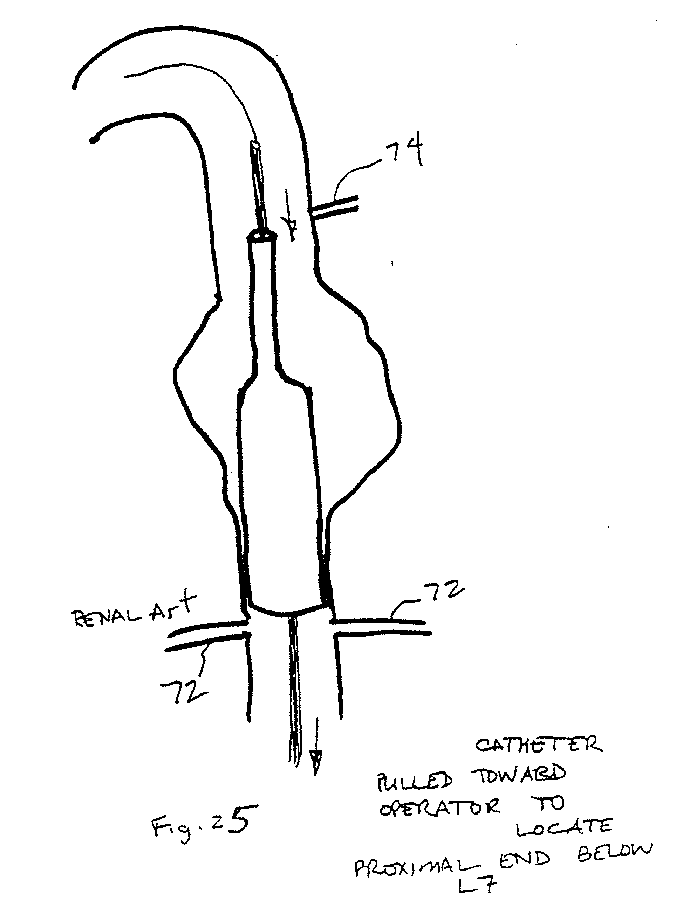

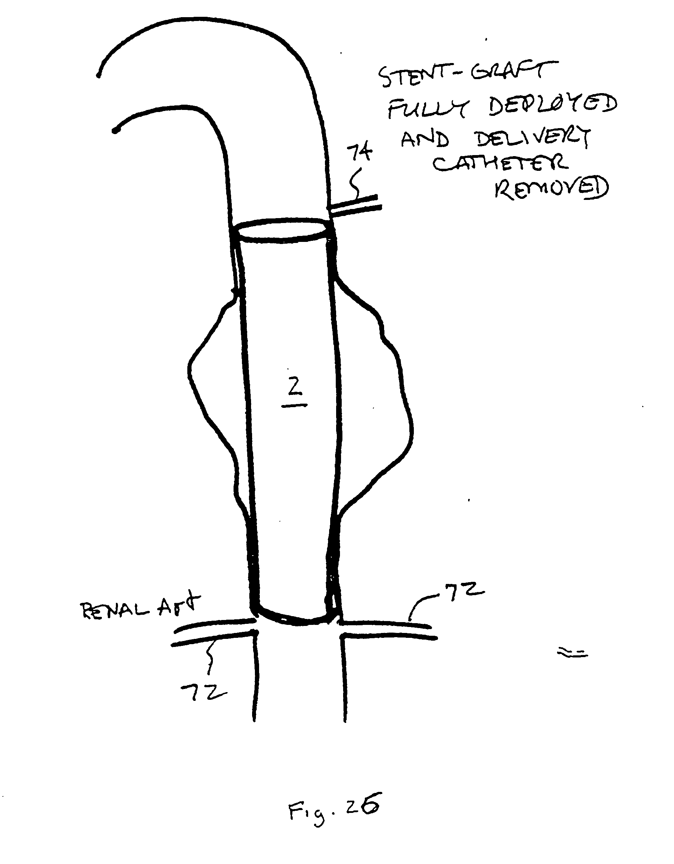

The physician estimates the length required, and chooses a device which is at least as long, and usually slightly longer than the estimated length. The stent-graft is inserted through an introducer as is conventional in the art. It is advanced until its distal end 2a is located as desired near the renal arteries (72) (Fig. 23). At this point, the proximal end of the stent-graft would be located at or past the T-7 artery (74). The stent-graft deployment is initiated slowly, distal to proximal (downstream to upstream') (Fig. 24) while watching the proximal end location on fluoroscopy. As needed, the delivery catheter 76, which is of conventional construction, is pulled toward the operator, shortening the stent-graft to keep the proximal end in the correct/desired location. This shortening can occur as long as the portion of the stent-graft being compressed is within the aneurysm 78. Once the proximal end is correctly located below the T-7 artery (Fig. 25), the stent-graft is fully deployed, and the delivery catheter is removed (Fig. 26).

The above is a detailed description of a particular embodiment of the invention. The full scope of the invention is set out in the claims that follow and their equivalents.

## Claims

1. A stent-graft (2) comprising:
a stent member having an inner surface and an outer surface (6);
a generally tubular graft member (4);
a coupling member (8) covering only a portion of at least one of the inner and outer surfaces of said stent member (6) that secures the stent member (6) and the graft member (4) together;
wherein said coupling member (8) is in the form of a ribbon.

2. A stent-graft (2) according to claim 1, wherein the stent member (6) comprises undulations (14).

3. A stent-graft (2) according to claim 1, wherein the stent member (6) is arranged in a helical configuration with multiple turns (12).

4. A stent-graft (2) according to claim 3, wherein a first helical turn is substantially in-phase with an adjacent second helical turn.

5. A stent-graft (2) according to claim 3, wherein apexes (16) and base portions (18) of the stent member (6) are configured so as not to restrain movement of one apex by an undulation in an adjacent turn.

6. A stent-graft (2) according to claim 3, wherein spacing between said turns (12) is uniform.

7. A stent-graft (2) according to claim 1, wherein one of said stent member (6) or said graft member (4) surrounds at least a portion of the other.

8. A stent-graft (2) according to claim 1, wherein said coupling member (8) is made from thermoplastic polymers.

9. A stent-graft (2) according to claim 1, wherein said coupling member is made from thermoplastic fluoropolymers.

10. A stent-graft (2) according to claim 1, wherein said coupling member (8) comprises expanded PTFE.

11. A stent-graft (2) according to claim 1, wherein said coupling member (8) is an FEP-coated porous expanded PTFE film.

12. A stent-graft (2) according to claim 1, wherein said stent member (6) comprises a tubular member.

13. A stent-graft (2) according to claim 1, wherein said stent member (6) comprises multiple tubular members.

14. A stent-graft (2) according to claim 1, wherein said stent member (6) comprises an expandable and an expanded configuration.

15. A stent-graft (2) according to claim 1, wherein said stent member (6) comprises a self-expanding stent.

16. A stent-graft (2) according to claim 1, wherein said stent-graft (2) has an expandable and an expanded configuration.

17. A stent-graft (2) according to claim 1, wherein said stent member (6) comprises a nickel-titanium alloy.

18. A stent-graft (2) according to claim 1, wherein said stent member (6) comprises nitinol.

19. A stent-graft (2) according to claim 1, wherein said graft member (4) comprises polymeric materials.

20. A stent-graft (2) according to claim 1, wherein said graft member (4) comprises expanded PTFE.

21. A stent-graft (2) according to claim 1, wherein said stent-graft (2) comprises radio-opaque fibers.

22. A stent-graft (2) according to claim 1, wherein said graft member (4) has an average thickness of less than or equal to about 0.15 mm.

23. A stent-graft (2) according to claim 1, wherein said coupling member (8) has an average thickness less than or equal to about 0.12 mm.

24. A stent-graft (2) according to claim 1, wherein said coupling member (8) covers a portion of the stent member (6).

25. A stent-graft (2) according to claim 1, wherein said coupling member (8) is arranged in a helical configuration with multiple turns (12).

26. A stent-graft (2) according to claim 25, wherein each of the multiple turns (12) of the coupling member (8) are spaced from an adjacent turn.

27. A stent-graft (2) according to claim 2, wherein said coupling member (8) is interwoven into a number of said undulations (14).

28. A stent-graft (2) according to claim 1, wherein said coupling member (8) comprises expanded PTFE.

29. A stent-graft (2) according to claim 2, wherein said coupling member (8) has a width less than or equal to about two-thirds the average amplitude, measured peak-to-peak, of a number of said undulations (14).

30. A stent-graft (2) according to claim 1, wherein said coupling member (8) covers only a portion of the stent member (6).

31. A stent-graft (2) according to claim 1, wherein said coupling member (8) having a generally flat surface is bonded to said graft member (4).

32. A stent-graft (2) according to claim 1, further comprising a linking member (20) disposed between adjacent turns (12) of the stent member (6).

33. A stent-graft (2) according to claim 1, further comprising a linking member (20) threaded between adjacent turns (12) to maintain undulations (14) in adjacent turns (12) generally in-phase therewith.

34. A stent-graft (2) according to claim 33, wherein the linking member (20) maintains a phased relationship of undulations (14) during compression and deployment, and during bending of the stent member (6).

35. A stent-graft (2) according to claim 1, wherein the stent member (6) is generally tubular and has inner and outer circumferences, said stent member (6) has a helical configuration with multiple helical turns (12) and multiple undulations (14), each undulation (14) having an apex (16);
said stent-graft (2) further comprising:
a linking member (20) threaded through adjacent apexes (16) in adjacent turns (12) to maintain undulations (14) in adjacent turns generally in-phase with one another;
said coupling member (8) being arranged and disposed to contact at least one of said inner and outer circumferences of said stent member (6) and forming multiple windings spaced from one another;
and wherein said tubular graft member (4) is disposed between said stent member (6) and said helically arranged coupling member (8) and including portions secured to said coupling member (8).

36. A stent-graft (2) according to claim 35, wherein said helically wound coupling member (8) is generally spaced from said apexes (16) and forms therewith respective openings, said apexes (16) being configured to permit unrestrained movement of said coupling member (20) within said openings.

## Patentansprüche

1. Stent-Transplantat (2), das aufweist:
ein Stentelement mit einer Innenfläche und einer Außenfläche (6);
ein im Allgemeinen rohrförmiges Transplantatelement (4);
ein Kopplungselement (8), das nur einen Abschnitt von mindestens einer der Innen- und der Außenfläche des Stentelementes (6) bedeckt, das das Stentelement (6) und das Transplantatelement (4) miteinander sichert;
wobei das Kopplungselement (8) in der Form eines Bandes vorliegt.

2. Stent-Transplantat (2) nach Anspruch 1, bei dem das Stentelement (6) Wellen (14) aufweist.

3. Stent-Transplantat (2) nach Anspruch 1, bei dem das Stentelement (6) in einer spiralförmigen Konfiguration mit mehreren Windungen (12) angeordnet ist.

4. Stent-Transplantat (2) nach Anspruch 3, bei dem eine erste spiralförmige Windung im Wesentlichen phasengleich mit einer benachbarten zweiten spiralförmigen Windung ist.

5. Stent-Transplantat (2) nach Anspruch 3, bei dem Scheitel (16) und Basisabschnitte (18) des Stentelementes (6) so ausgebildet sind, dass sie nicht die Bewegung eines Scheitels durch eine Welle in einer benachbarten Windung einschränken.

6. Stent-Transplantat (2) nach Anspruch 3, bei dem der Abstand zwischen den Windungen (12) gleichmäßig ist.

7. Stent-Transplantat (2) nach Anspruch 1, bei dem das Stentelement (6) oder das Transplantatelement (4) mindestens einen Abschnitt des anderen umgibt.

8. Stent-Transplantat (2) nach Anspruch 1, bei dem das Kopplungselement (8) aus thermoplastischen Polymeren besteht.

9. Stent-Transplantat (2) nach Anspruch 1, bei dem das Kopplungselement aus thermoplastischen Fluorpolymeren besteht.

10. Stent-Transplantat (2) nach Anspruch 1, bei dem das Kopplungselement (8) expandiertes PTFE aufweist.

11. Stent-Transplantat (2) nach Anspruch 1, bei dem das Kopplungselement (8) eine mit FEP beschichtete poröse expandierte PTFE-Folie ist.

12. Stent-Transplantat (2) nach Anspruch 1, bei dem das Stentelement (6) ein rohrförmiges Element aufweist.

13. Stent-Transplantat (2) nach Anspruch 1, bei dem das Stentelement (6) mehrere rohrförmige Elemente aufweist.

14. Stent-Transplantat (2) nach Anspruch 1, bei dem das Stentelement (6) eine expandierbare und eine expandierte Konfiguration aufweist.

15. Stent-Transplantat (2) nach Anspruch 1, bei dem das Stentelement (6) einen selbstexpandierenden Stent aufweist.

16. Stent-Transplantat (2) nach Anspruch 1, bei dem das Stent-Transplantat (2) eine expandierbare und eine expandierte Konfiguration aufweist.

17. Stent-Transplantat (2) nach Anspruch 1, bei dem das Stentelement (6) eine Nickel-Titan-Legierung aufweist.

18. Stent-Transplantat (2) nach Anspruch 1, bei dem das Stentelement (6) Nitinol aufweist.

19. Stent-Transplantat (2) nach Anspruch 1, bei dem das Transplantatelement (4) polymere Materialien aufweist.

20. Stent-Transplantat (2) nach Anspruch 1, bei dem das Transplantatelement (4) expandiertes PTFE aufweist.

21. Stent-Transplantat (2) nach Anspruch 1, bei dem das Stent-Transplantat (2) röntgensichtbare Fasern aufweist.

22. Stent-Transplantat (2) nach Anspruch 1, bei dem das Transplantatelement (4) eine mittlere Dicke von weniger als oder gleich etwa 0,2 mm (0,006 in.) aufweist.

23. Stent-Transplantat (2) nach Anspruch 1, bei dem das Kopplungselement (8) eine mittlere Dicke von weniger als oder gleich etwa 0,1 mm (0,005 in.) aufweist.

24. Stent-Transplantat (2) nach Anspruch 1, bei dem das Kopplungselement (8) einen Abschnitt des Stentelementes (6) bedeckt.

25. Stent-Transplantat (2) nach Anspruch 1, bei dem das Kopplungselement (8) in einer spiralförmigen Konfiguration mit mehreren Windungen (12) angeordnet ist.

26. Stent-Transplantat (2) nach Anspruch 25, bei dem eine jede der mehreren Windungen (12) des Kopplungselementes (8) von einer benachbarten Windung beabstandet ist.

27. Stent-Transplantat (2) nach Anspruch 2, bei dem das Kopplungselement (8) in einer Anzahl der Wellen (14) verflochten ist.

28. Stent-Transplantat (2) nach Anspruch 1, bei dem das Kopplungselement (8) expandiertes PTFE aufweist.

29. Stent-Transplantat (2) nach Anspruch 2, bei dem das Kopplungselement (8) eine Breite kleiner als oder gleich etwa zwei Drittel der mittleren Amplitude, gemessen von Spitze zu Spitze, einer Anzahl der Wellen (14) aufweist.

30. Stent-Transplantat (2) nach Anspruch 1, bei dem das Kopplungselement (8) nur einen Abschnitt des Stentelementes (6) bedeckt.

31. Stent-Transplantat (2) nach Anspruch 1, bei dem das Kopplungselement (8), das eine im Allgemeinen flache Fläche aufweist, am Transplantatelement (4) gebunden ist.

32. Stent-Transplantat (2) nach Anspruch 1, das außerdem ein Verbindungselement (20) aufweist, das zwischen benachbarten Windungen (12) des Stentelementes (6) angeordnet ist.

33. Stent-Transplantat (2) nach Anspruch 1, das außerdem ein Verbindungselement (20) aufweist, das zwischen benachbarten Windungen (12) eingefädelt wird, um Wellen (14) in benachbarten Windungen (12) im Allgemeinen phasengleich damit zu halten.

34. Stent-Transplantat (2) nach Anspruch 33, bei dem das Verbindungselement (20) eine Phasenbeziehung der Wellen (14) während des Zusammendrückens und Entfaltens und während des Biegens des Stentelementes (6) beibehält.

35. Stent-Transplantat (2) nach Anspruch 1, bei dem das Stentelement (6) im Allgemeinen rohrförmig ist und einen Innen- und einen Außenumfang aufweist, wobei das Stentelement (6) eine spiralförmige Konfiguration mit mehreren spiralförmigen Windungen (12) und mehreren Wellen (14) aufweist, wobei eine jede Welle (14) einen Scheitel (16) aufweist;
wobei das Stent-Transplantat (2) außerdem aufweist:
ein Verbindungselement (20), das durch benachbarte Scheitel (16) in benachbarten Windungen (12) eingefädelt wird, um Wellen (14) in benachbarten Windungen im Allgemeinen phasengleich miteinander zu halten;
wobei das Kopplungselement (8) eingerichtet und angeordnet ist, um mindestens einen des Innen- und des Außenumfanges des Stentelementes (6) zu berühren, und mehrere Windungen bildet, die voneinander beabstandet sind; und
wobei das rohrförmige Transplantatelement (4) zwischen dem Stentelement (6) und dem spiralförmig angeordneten Kopplungselement (8) angeordnet ist und Abschnitte umfasst, die am Kopplungselement (8) gesichert sind.

36. Stent-Transplantat (2) nach Anspruch 35, bei dem das spiralförmig gewickelte Kopplungselement (8) im Allgemeinen von den Scheiteln (16) beabstandet ist und damit entsprechende Öffnungen bildet, wobei die Scheitel (16) ausgebildet sind, um eine uneingeschränkte Bewegung des Kopplungselementes (20) innerhalb der Öffnungen zu gestatten.

## Revendications

1. Greffe à stent (2), comprenant:
un élément de stent, comportant une surface interne et une surface externe (6);
un élément de greffe généralement tubulaire (4);
un élément d'accouplement (8) recouvrant uniquement une partie d'au moins une des surfaces interne et externe dudit élément de stent (6), assurant l'assemblage de l'élément de stent (6) et de l'élément de greffe (4) ;
ledit élément d'accouplement (8) ayant la forme d'un ruban.

2. Greffe à stent (2) selon la revendication 1, dans laquelle l'élément de stent (6) comprend des ondulations (14).

3. Greffe à stent (2) selon la revendication 1, dans laquelle l'élément de stent (6) est agencé dans une configuration hélicoïdale comportant des spires multiples (12).

4. Greffe à stent (2) selon la revendication 3, dans laquelle une première spire hélicoïdale est pratiquement en phase avec une deuxième spire hélicoïdale adjacente.

5. Greffe à stent (2) selon la revendication 3, dans laquelle les sommets (16) et les parties de base (18) de l'élément de stent (6) sont configurés de sorte à ne pas limiter le déplacement d'un sommet par une ondulation dans une spire adjacente.

6. Greffe à stent (2) selon la revendication 3, dans laquelle l'espacement entre les spires (12) est uniforme.

7. Greffe à stent (2) selon la revendication 1, dans laquelle un élément, ledit élément de stent (6) ou ledit élément de greffe (4) entoure au moins une partie de l'autre élément.

8. Greffe à stent (2) selon la revendication 1, dans laquelle ledit élément d'accouplement (8) est composé de polymères thermoplastiques.

9. Greffe à stent (2) selon la revendication 1, dans laquelle ledit élément d'accouplement est composé de polymères fluorés thermoplastiques.

10. Greffe à stent (2) selon la revendication 1, dans laquelle ledit élément d'accouplement (8) est composé de PTFE expansé.

11. Greffe à stent (2) selon la revendication 1, dans laquelle ledit élément d'accouplement (8) est constitué par un film de PYFE expansé poreux recouvert de FEP.

12. Greffe à stent (2) selon la revendication 1, dans laquelle ledit élément de stent (6) est constitué par un élément tubulaire.

13. Greffe à stent (2) selon la revendication 1, dans laquelle ledit élément de stent (6) est constitué par de multiples éléments tubulaires.

14. Greffe à stent (2) selon la revendication 1, dans laquelle ledit élément de stent (6) a une configuration extensible et une configuration étendue.

15. Greffe à stent (2) selon la revendication 1, dans laquelle ledit élément de stent (6) est constitué par un stent à extension automatique.

16. Greffe à stent (2) selon la revendication 1, dans laquelle ladite greffe à stent (2) a une configuration extensible et une configuration étendue.

17. Greffe à stent (2) selon la revendication 1, dans laquelle ledit élément de stent (6) est composé d'un alliage de titane au nickel.

18. Greffe à stent (2) selon la revendication 1, dans laquelle ledit élément de stent (6) est composé de nitinol.

19. Greffe à stent (2) selon la revendication 1, dans laquelle ledit élément de greffe (4) est composé de matériaux polymères.

20. Greffe à stent (2) selon la revendication 1, dans laquelle ledit élément de greffe (4) est composé de PTFE expansé.

21. Greffe à stent (2) selon la revendication 1, dans laquelle ladite greffe à stent (2) comprend des fibres radio-opaques.

22. Greffe à stent (2) selon la revendication 1, dans laquelle ledit élément de greffe (4) a une épaisseur moyenne inférieure ou égale à environ à 0,2 mm (0,006 pouce).

23. Greffe à stent (2) selon la revendication 1, dans laquelle ledit élément d'accouplement (8) a une épaisseur moyenne inférieure ou égale à environ à 0,1 mm (0,005 pouce).

24. Greffe à stent (2) selon la revendication 1, dans laquelle ledit élément d'accouplement (8) recouvre une partie de l'élément de stent (6).

25. Greffe à stent (2) selon la revendication 1, dans laquelle ledit élément d'accouplement (8) est agencé dans une configuration hélicoïdale, comportant de multiples spires (12).

26. Greffe à stent (2) selon la revendication 25, dans laquelle chacune des multiples spires (12) de l'élément d'accouplement (8) est espacée d'une spire adjacente.

27. Greffe à stent (2) selon la revendication 2, dans laquelle ledit élément d'accouplement (8) est entrelacé dans plusieurs desdites ondulations (14).

28. Greffe à stent (2) selon la revendication 1, dans laquelle ledit élément d'accouplement (8) est composé de PTFE expansé.

29. Greffe à stent (2) selon la revendication 2, dans laquelle ledit élément d'accouplement (8) a une largeur représentant moins d'environ les deux tiers de l'amplitude moyenne ou égale à celle-ci, mesurée entre les crêtes, de plusieurs desdites ondulations (14).

30. Greffe à stent (2) selon la revendication 1, dans laquelle ledit élément d'accouplement (8) ne recouvre qu'une partie de l'élément de stent (6).

31. Greffe à stent (2) selon la revendication 1, dans laquelle ledit élément d'accouplement (8), comportant une surface généralement plate, est reliée audit élément de greffe (4).

32. Greffe à stent (2) selon la revendication 1, comprenant en outre un élément de liaison (20) agencé entre des spires adjacentes (12) de l'élément de stent (6).

33. Greffe à stent (2) selon la revendication 1, comprenant en outre un élément de liaison (20) fileté entre des spires adjacentes (12) pour maintenir les ondulations (14) dans les spires adjacentes (12) généralement en phase avec celles-ci.

34. Greffe à stent (2) selon la revendication 33, dans laquelle l'élément de liaison (20) maintient une relation en phase des ondulations (14) au cours de la compression et du déploiement et au cours de la flexion de l'élément de stent (6).

35. Greffe à stent (2) selon la revendication 1, dans laquelle l'élément de stent (6) est généralement tubulaire et comporte des circonférences interne et externe, ledit élément de stent (6) ayant une configuration hélicoïdale avec de multiples spires hélicoïdales (12) et de multiples ondulations (14), chaque ondulation (14) comportant un sommet (16) ;
ladite greffe à stent (2) comprenant en outre :
un élément de liaison (20) fileté à travers les sommets adjacents (16) dans les spires adjacentes (12) pour maintenir les ondulations (14) dans les spires adjacentes généralement en phase les unes avec les autres ;
ledit élément d'accouplement (8) étant agencé et positionné de sorte à contacter au moins une desdites circonférences interne et externe dudit élément de stent (6) et formant de multiples enroulements espacés les uns des autres ;
ledit élément de greffe tubulaire (4) étant agencé entre ledit élément de stent (6) et ledit élément d'accouplement à agencement hélicoïdal (8) et englobant des parties fixées sur ledit élément d'accouplement (8).

36. Greffe à stent (2) selon la revendication 35, dans laquelle ledit élément d'accouplement à enroulement hélicoïdal (8) est généralement espacé desdits sommets (16) et forme avec ceux-ci des ouvertures respectives, lesdits sommets (16) étant configurés de sorte à permettre un déplacement illimité dudit élément d'accouplement (20) dans lesdites ouvertures.
